# EUROPEAN PATENT SPECIFICATION

(11) **EP 2 080 024 B1**
(45) Date of publication and mention of the grant of the patent: **06.10.2010**
(21) Application number: 07834636.8
(22) Date of filing: 16.10.2007
(51) Int. Cl.: G01N 33/50, G01N 33/569

(54) **CROSS-SPOT**
CROSS-SPOT
TEST "CROSS-SPOT"

(30) Priority: 16.10.2006 EP 06122334; 16.10.2006 US 851765 P
(43) Date of publication of application: 22.07.2009
(73) Proprietor: Thijsen, Steven Frederik Theodoor, 3708 BH Zeist (NL); Bossink, Ailko Wim Jan, 3723 ER Bilthoven (NL)
(72) Inventor: BOSSINK, Ailko Wim Jan, 3723 ER Bilthoven (NL)
(74) Representative: Swinkels, Bart Willem
(86) International application number: PCT/NL2007/050498
(87) International publication number: WO 2008/048097

(56) References cited:
- SMITH S M ET AL: "Human CD8+ CTL specific for the Mycobacterial major secreted antigen 85A" JOURNAL OF IMMUNOLOGY, THE WILLIAMS AND WILKINS CO. BALTIMORE, US, vol. 165, 2000, pages 7088-7095, XP002173902 ISSN: 0022-1767
- HERR W ET AL: "Identification of naturally processed and HLA-presented Epstein-Barr virus peptides recognized by CD4(+) or CD8(+) T lymphocytes from human blood." PROCEEDINGS OF THE NATIONAL ACADEMY OF SCIENCES OF THE UNITED STATES OF AMERICA. 12 OCT 1999, vol. 96, no. 21, 12 October 1999 (1999-10-12), pages 12033-12038, XP002411830 ISSN: 0027-8424
- JAFARI CLAUDIA ET AL: "Rapid diagnosis of smear-negative tuberculosis by bronchoalveolar lavage enzyme-linked immunospot." AMERICAN JOURNAL OF RESPIRATORY AND CRITICAL CARE MEDICINE 1 NOV 2006, vol. 174, no. 9, 20 July 2006 (2006-07-20), pages 1048-1054, XP009076297 ISSN: 1073-449X

## Description

### Field of the invention

The invention relates to an *in vitro* method for measuring the presence of an antigen indicative for the presence of an infectious agent, and preferably a medical condition or disease in a sample using an immune effector cell capable of producing an immune effector molecule following stimulation by an antigen.

### Background of the invention

Several diagnostic assays are already known allowing the detection of a Cell-Mediated Immune response (CMI). Current methods for detecting CMI responses include skin tests measuring both immediate and delayed type hypersensitivity, lymphocyte proliferation assays and measurement of cytokines produced by purified mononuclear cells cultured with antigen. Most *in vitro* methods for detecting CMI responses involve the purification of lymphocytes from whole blood, culturing these lymphocytes with a known antigen for periods from 12 hours to 6 days and then detecting T-cell reactivity to the known antigen. Older, established methods, such as the proliferation assay, use the uptake of radioactive isotopes by dividing T-cells as a marker for CMI reactivity. More recently, techniques such as a single cell assay (ELISpot which stands for enzyme-linked immunospot assay) have been used to detect the number of T-cells producing certain cytokines in response to the antigenic stimulation. Alternatively, B-cells can be used instead of T-cells as immune effector cells in which event the immune effector molecules are immunoglobulins (Quan Chao et al, J. Clin. Microbiol., 2006, p3160-3166). Despite the existence of above-mentioned assays for measuring CMI responsiveness, the practical limitations of getting enough lymphocytes from subjects to be tested and ensuring they would still be able to respond to antigen stimulation precluded large scale adoption of these assays in standard and routine medical practice in the diagnosis of infectious disease. Furthermore, obtaining large volumes of blood is especially difficult in young children and often an argument to not perform the test. Thirdly, obtaining enough lymphocytes from other body fluids for use in an ELISpot is sometimes impossible. In addition, function of immune effector cells can be compromised for example by the use of biological response modifiers such as Tumor Necrosis Factor alfa inhibitors.

Therefore, there is still a need for diagnostic methods that do not possess all the drawbacks of known methods.

### Description of the invention

### Method

In a first aspect, the invention relates to an *in vitro* method for measuring the presence or elevation in the level of an antigen in a sample, said antigen being indicative for the presence of an infectious agent, and preferably a medical condition or disease. This method comprises the following steps:
(i) contacting an immune effector cell with a sample, wherein an immune effector cell is capable of producing an immune effector molecule following stimulation by an antigen,
(ii) measuring the presence or elevation in the level of an immune effector molecule, wherein the presence or elevation in the level of said immune effector molecule is indicative of the presence of an antigen in a sample.

### First step

### Sample

A sample may be of any kind as long as it is desirable to establish whether or not the sample contains a given antigen indicative for the presence of an infectious agent, and preferably a medical condition or disease. A sample may be in a liquid or solid state. Depending on its state, the skilled person would know how to prepare suitable preparations of the sample in order to be contacted with the immune cells. A sample may originate from food, feed, coating for medicinal apparatus, drugs. In a more preferred embodiment, a sample is a biological sample. Preferred biological samples are taken from a subject. A subject is extensively defined in next paragraph. Briefly, a subject may be a human or animal. Preferably, a sample is a body fluid from a subject. Preferred human or animal fluids are selected from the following list: urine, faeces, blood, lymph fluid, cerebral fluid, tissue fluid, respiratory fluid including nasal and pulmonary fluid or fluids obtained by nasal or pulmonary lavage, pleural fluid and synovial fluid.

An antigen that may be detected in a sample using the method of the invention is not a specific antigen. In a preferred method of the invention, the method is used for establishing the presence of an infectious agent and preferably a medical condition or a disease. In this preferred method, an infectious agent, the medical condition or the disease is associated with the presence of an antigen in a sample taken from a subject.

### Subject

Reference to a "subject" includes a human or non-human species including primates, livestock animals (e.g. sheep, cows, pigs, horses, donkey, goats), laboratory test animals (e.g. mice, rats, rabbits, guinea pigs, hamsters), companion animals (e.g. dogs, cats), avian species (e.g. poultry birds, aviary birds), reptiles and amphibians. The present invention has applicability, therefore, in human medicine as well as in livestock and veterinary and wild life applications. Most preferably, however, a subject is a human and the method has applications in screening for responsiveness to any pathogenic microorganisms, bacteria, viruses, parasites, and prion, potential for development or monitoring autoimmune conditions and for monitoring a subject's response to oncological treatment.

### Immune effector cell

An immune effector cells used in the method of the invention is capable of producing an immune effector molecule following stimulation (CMI response) by a given antigen being indicative for the presence of an infectious agent and preferably a medical condition or disease. Typically, these cells are representative from a subject known to have been in contact with said antigen. In this context "subject" has the same meaning as defined earlier. These immune effector cells include cells such as lymphocytes including natural killer (NK) cells, T-cells, (CD4⁺ and/or CD8⁺ cells), B-cells, macrophages and monocytes, dendritic cells or any other cell which is capable of producing an immune effector molecule in response to direct or indirect antigen stimulation. Conveniently, an immune effector cell is a lymphocyte. In a preferred embodiment, any immune effector cell is used wherein it has first been established this immune effector cell is able to give a CMI for a given antigen indicative for the presence of an infectious agent, and preferably a medical condition or disease.

Preparation of an immune effector cell or an antigen-specific T-cell line Alternatively, if no immune effector cell is available to be used in the method of the invention, an immune effector cell may be first prepared by contacting *in vitro, in vivo* or *ex vivo* naive immune cell with a given antigen, and/or infectious agent. CMI responsive immune effector cells are subsequently identified and stored. To establish long-term, antigen-specific T-cell lines, blood is first isolated. Subsequently, Peripheral Blood mononuclear cells (PBMC)s are purified therefrom via a classical Ficoll separation step. These purified PBMCs can be stimulated with specific antigen upon which T-cell clones can be immortalised with for example a virus-based retroviral vector carrying an immortalizing gene. This approach will generate immortalized antigen-specific IL-2-dependent T-cell lines with HLA-restricted, antigen-specific responsiveness as described by Barsov et al. (Barsov et al, imm letters 2006 May 15;105(1):26-37. Epub 2005 Dec 21). These cell lines can be stored in the vapor phase of liquid nitrogen after controlled freezing in 20% serum albumin and 10% DMSO. These cells could be later used in the method of the invention.

An immune effector cell is preferably suited for the method of the invention when it has first been found positive in a classical ELISA or ELISpot immunoassay(for example T-SPOT™. *TB* from Oxford Immunotec Ltd.) as defined herein. Accordingly, in a preferred embodiment, to detect the presence of an antigen associated with a mycobacterial disease, a T cell known to be T-SPOT™.TB positive is used.

An immune effector cell is found positive in an ELISA assay or ELISpot assay when a cell is found to give a specific CMI response for a given antigen.

Preferably, an immune effector cell is found to give a specific CMI response for a given antigen in an ELISA assay when an elevation in the level of an immune effector molecule of at least 10% is measured compared to the level of the immune effector molecule measured in a negative control. More preferably, the elevation is of at least 30%, even more preferably of at least 50%, even more preferably of at least 70%, even more preferably of at least 100%, even more preferably of at least 200%, even more preferably of at least 500%, and most preferably of at least 1000%. Negative control is typically when an immune effector cell is not contacted with an antigen.

Alternatively or in combination with previous preferred embodiment, an immune effector cell is found to give a specific CMI response for a given antigen in an ELISpot assay when at least 5% more spots are found compared to the number of spots present in a negative control. Negative control is typically when an immune effector cell is not contacted with an antigen. More preferably, the elevation is of at least 10% more spots, even more preferably of at least 20%, even more preferably of at least 30%, even more preferably of at least 40%, even more preferably of at least 50%, even more preferably of at least 70%, and most preferably of at least 100%.

In a preferred method, an immune effector cell used is epitope specific for example EBNA 1 specific T-cell clones (Cells. J Immunol. 2006 Sep 15;177(6):3746-56. Taylor GS et al). These cells are preferably used in the method of the invention wherein the antigen is EBV.

In another preferred method, an immune effector cell used is a tuberculosis (TB) specific polyclonal or monoclonal T-cell. More preferably, such an immune effector cell is specific for at least one of the following antigens from mycobacterium: ESAT-6 (Early Secreted Antigenic Target -6, 6kDa) and CFP-10 (Culture Filtrate Protein-10, 10 kDa). Such immune effector cells are available via David Lewinsohn (Oregon Health Science University, R&D 11, Portland VA Medical Center, 3710 SW US Veterans Road, Portland, OR, USA, lewinsod@ohsu.edu).

In a preferred method, at least one type of immune effector cells is used; each type being capable of producing a given immune effector molecule following stimulation by the antigen. In this preferred method, the presence of an antigen is indicated by the presence or elevation in the level of at least one immune effector molecule. More preferably, T and B cells are used as an immune effector cell and IFNγ and an immunoglobulin of the IgM, IgE, IgG or IgA classes are used as an immune effector molecule.

### Immune effector molecule

An immune effector molecule(s) may be any of a range of molecules which are produced in response to cell activation or stimulation by an antigen. Although an interferon (IFN) such as IFN-y is a particularly useful immune effector molecule produced by immune T cells, others include a range of cytokines such as interleukins (IL), e.g. IL-2,IL-4, IL-10 or IL-12, tumor necrosis factor alpha (TNF-α), a colony stimulating factor (CSF) such as granulocyte (G)-CSF or granulocyte macrophage (GM)-CSF amongst many others such as complement or components in the complement pathway. Alternatively, if an immune cell used is a B cell, an immunoglobulin may be an immune effector molecule. In a more preferred embodiment, an immunoglobulin is selected from the group consisting of the IgM, IgG, IgE and IgA immunoglobulin classes. In an even more preferred embodiment, an immune effector molecule is selected from the group consisting of: IFNγ and an immunoglobulin. In an even more preferred embodiment, an immunoglobulin is selected from the group consisting of the IgM, IgG, IgE and IgA immunoglobulin classes. Accordingly, in a preferred embodiment, the present invention provides an *in vitro* method for measuring the presence or elevation in the level of an antigen in a sample, said antigen being indicative for the presence of an infectious agent, and preferably a medical condition or disease. This method comprises the following steps:
(i) contacting an immune effector cell with a sample, wherein the immune effector cells are capable of producing IFNγ following stimulation by the antigen,
(ii) measuring the presence or elevation in the level of IFNγ, wherein the presence or elevation in the level of IFNγ is indicative of the presence of an antigen in the sample.

Alternatively or in combination with previous preferred embodiment, the present invention provides an *in vitro* method for measuring the presence or elevation in the level of an antigen in a sample, said antigen being indicative for the presence of an infectious agent, and preferably a medical condition or disease. This method comprises the following steps:
(i) contacting an immune effector cell with a sample, wherein the immune effector cells are capable of producing an immunoglobulin following stimulation by the antigen,
(ii)measuring the presence or elevation in the level of an immunoglobulin ,wherein the presence or elevation of the immunoglobulin in the level is indicative of the presence of an antigen in the sample.

In this embodiment, it is further preferred that the immunoglobulin is selected from the group consisting of the IgM, IgG, IgE and IgA immunoglobulin classes.

The duration of the contact step could vary depending on the method used for measuring the presence or elevation in the level of the immune effector molecule. Typically, the duration of the contact step may be from 5 to 50 hours, more preferably 5 to 40 hours and even more preferably 8 to 24 hours . Typically, 10⁵ to 10⁷ immune effector cells, preferably 5x10⁵ to 10⁶ immune effector cells are contacted with the sample. Typically, the contact step is carried out in a suitable medium for immune effector cells.

### Infectious agent, disease

The ability to measure the presence or elevation in the level of an antigen is important for deducting the presence of an infectious agent, and preferably a medical condition or disease in a sample. An infectious agent may be any known pathogenic agent such as a microorganism or virus or parasite for which specific reactive immune cells are available or can be prepared as earlier defined (see paragraph entitled "preparation of immune effector cells"). Alternatively, an antigen may be an endogenous antigen and its detection by the immune cell would be seen as a marker for an autoimmune, allergic disease or cancer.

Autoimmune diseases contemplated herein include *inter alia* Alopecia Areata, Ankylosing Spondylitis, Asthma, Extrinsic Allergic Alveolitis, Anaphylaxis, Atopic Dermatitis, Drug Allergy, Occupational Allergy, Allergic Contact Dermatitis Antiphospholipid Syndrome, Autoimmune Addison's Disease, Multiple Sclerosis, Autoimmune disease of the adrenal gland, Autoimmune Hemolytic Anemia, Autoimmune Hepatitis, Autoimmune oophoritis and orchitis, Behcet's Disease, Bullous Pemphigoid, Cardiomyopathy, Celiac Sprue-Dermatitis, Chronic Fatigue Syndrome (CFIDS), Chronic Inflam. Demyelinating Chronic Inflam. Polyneuropathy, Churg-Strauss Syndrome, Cicatricial Pemphigoid, CREST Syndrome, Cold Agglutinin Disease, Crohn's Disease, Conjunctivitis, Dermatitis herpetiformis, Discoid Lupus, Essential Mixed Cryoglobulinemia, Fibromyalgia, Glomeralonephritis, Grave's Disease, Guillain-Barre, Hashimoto's Thyroiditis, Idiopathic Pulmonary Fibrosis, Idiopathic Thrombocytopenia Purpura (ITP), IgA Nephropathy, Insulin Dependent Diabetes (Type 1), Lichen Planus, Lupus, Meniere's Disease, Mixed Connective Tissue Disease, Multiple sclerosis, Myasthenia Gravis, Myocarditis, Pemphigus Vulgaris, Pernicious Anemia, Polyarteritis Nodosa, Polychondritis, Polyglancular Syndromes, Polymyalgia Rheumatica, Polymyositis and Dermatomyositis, Primary Agammaglobulinemia, Primary Biliary Cirrhosis, Psoriasis, Raynaud's Phenomenon, Reiter's Syndrome, Rheumatic Fever, Rheumatoid Arthritis, Rhinitis, Sarcoidosis, Scleroderma, Sjogren's Syndrome, Stiff-Man Syndrome, Systemic lupus erythematosus, Takayasu Arteritis, Temporal Arteritis/Giant Cell Arteritis, Ulcerative Colitis, Uveitis, Urticaria, Vasculitis and Vitiligo.

Cancers contemplated herein include: a group of diseases and disorders that are characterized by uncontrolled cellular growth (e.g. formation of tumor) without any differentiation of those cells into specialized and different cells. Such diseases and disorders include ABL1 protooncogene, ADDS Related Cancers, Acoustic Neuroma, Acute Lymphocytic Leukaemia, Acute Myeloid Leukaemia, Adenocystic carcinoma, Adrenocortical Cancer, Agnogenic myeloid metaplasia, Alopecia, Alveolar soft-part sarcoma, Anal cancer, Angiosarcoma, Aplastic Anaemia, Astrocytoma, Ataxia-telangiectasia, Basal Cell Carcinoma (Skin), Bladder Cancer, Bone Cancers, Bowel cancer, Brain Stem Glioma, Brain and CNS Tumours, Breast Cancer, CNS tumours, Carcinoid Tumours, Cervical Cancer, Childhood Brain Tumours, Childhood Cancer, Childhood Leukaemia, Childhood Soft Tissue Sarcoma, Chondrosarcoma, Choriocarcinoma, Chronic Lymphocytic Leukaemia, Chronic Myeloid Leukaemia, Colorectal Cancers, Cutaneous T-Cell Lymphoma, Dermatofibrosarcoma-protuberans, Desmoplastic-Small-Round-Cell-Tumour, Ductal Carcinoma, Endocrine Cancers, Endometrial Cancer, Ependymoma, Esophageal Cancer, Ewing's Sarcoma, Extra-Hepatic Bile Duct Cancer, Eye Cancer, Eye: Melanoma, Retinoblastoma, Fallopian Tube cancer, Fanconi Anaemia, Fibrosarcoma, Gall Bladder Cancer, Gastric Cancer, Gastrointestinal Cancers, Gastrointestinal-Carcinoid-Tumour, Genitourinary Cancers, Germ Cell Tumours, Gestational-Trophoblastic-Disease, Glioma, Gynaecological Cancers, Haematological Malignancies, Hairy Cell Leukaemia, Head and Neck Cancer, Hepatocellular Cancer, Hereditary Breast Cancer, Histiocytosis, Hodgkin's Disease, Human Papillomavirus, Hydatidiform mole, Hypopharynx Cancer, IntraOcular Melanoma, Islet cell cancer, Kaposi's sarcoma, Kidney Cancer, Langerhan's-Cell-Histiocytosis, Laryngeal Cancer, Leiomyosarcoma, Leukaemia, Li-Fraumeni Syndrome, Lip Cancer, Liposarcoma, Liver Cancer, Lung Cancer, Lymphedema,Lymphoma, Hodgkin's Lymphoma, Non-Hodgkin's Lymphoma, Male Breast Cancer, Malignant-Rhabdoid-Tumour-of Kidney, Medulloblastoma, Melanoma, Merkel Cell Cancer, Mesothelioma, Metastatic Cancer, Mouth Cancer, Multiple Endocrine Neoplasia, Mycosis Fungoides, Myelodysplastic Syndromes, Myeloma, Myeloproliferative Disorders, Nasal Cancer, Nasopharyngeal Cancer, Nephroblastoma, Neuroblastoma, Neurofibromatosis, Nijmegen Breakage Syndrome, Non-Melanoma Skin Cancer, Non-Small-Cell-Lung-Cancer (NSCLC), Ocular Cancers, Oesophageal Cancer, Oral cavity Cancer, Oropharynx Cancer, Osteosarcoma, Ostomy Ovarian Cancer, Pancreas Cancer, Paranasal Cancer, Parathyroid Cancer, Parotid Gland Cancer, Penile Cancer, Peripheral-Neuroectodermal-Tumours, Pituitary Cancer, Polycythemia vera, Prostate Cancer, Rare-cancers-and-associated-disorders, Renal Cell Carcinoma, Retinoblastoma, Rhabdomyosarcoma, Rothmund-Thomson Syndrome, Salivary Gland Cancer, Sarcoma, Schwannoma, Sezary syndrome, Skin Cancer, Small Cell Lung Cancer (SCLC), Small Intestine Cancer, Soft Tissue Sarcoma, Spinal Cord Tumours, Squamous-Cell-Carcinoma-(skin), Stomach Cancer, Synovial sarcoma, Testicular Cancer, Thymus Cancer, Thyroid Cancer, Transitional-Cell-Cancer-(bladder),Transitional-Cell-Cancer-(renal-pelvis-/-ureter), Trophoblastic Cancer, Urethral Cancer, Urinary System Cancer, Uroplakins, Uterine sarcoma, Uterus Cancer, Vaginal Cancer, Vulva Cancer, Waldenstrom's-Macroglobulinemia, Wihns' Tumour. Preferably, the cancer may be detected or measured by an immune cell able to detect malignancy specific epitopes for example derived from gene translocation and subsequent fused proteins such as BCR-ABL in chronic myeloid leukemia.

An infectious agent may give an inflammatory disease. Examples of inflammatory disease conditions contemplated by the present invention include but are not limited to those disease and disorders which result in a response of redness, swelling, pain, and a feeling of heat in certain areas that is meant to protect tissues affected by injury or disease. Inflammatory diseases which may be diagnosed using the methods of the present invention, include, without being limited to, acne, angina, arthritis, aspiration pneumonia, empyema, gastroenteritis, inflammation, intestinal flu, NEC, necrotizing enterocolitis, pelvic inflammatory disease, pharyngitis, pleurisy, raw throat, redness, rubor, sore throat, stomach flu and urinary tract infections, Chronic Inflammatory Demyelinating Polyneuropathy, Chronic Inflammatory Demyelinating Polyradiculoneuropathy.

In a preferred method, an infectious agent is selected from the following list: bacterium, virus, fungus, parasite, prion. More preferably, a bacterium is a mycobacterium. According to another more preferred embodiment, a virus is a herpes virus more particular Epstein Barr Virus (EBV) and Cytomegalo Virus (CMV). According to another more preferred embodiment, other virus include for example Human Herpes virus 6 (HHV6), Human Herpes Virus 8 (HHV8), parvovirus, Hepatitis B virus (HBV), Hepatitis C virus (HCV), Human immunodeficiency virus (HIV).

### Second step

In the second step of the method of the invention, the presence or elevation in the level of an immune effector molecule is measured, wherein the presence or elevation in the level of said immune effector molecule is indicative of the presence of an antigen in the sample. Depending on the identity of the immune effector molecule, the type of sample, the identity of the antigen and/or the identity of the infectious agent, the skilled person will know which amount or elevation in the level of an immune effector molecule will be equivalent with the development of a disease associated with the presence of the antigen. The measurement of the immune effector molecule may be made at the protein or nucleic acid levels. Consequently, reference to "presence or level of said immune effector molecule" includes direct and indirect data. For example, high levels of IFNγ mRNA is indirect data showing increased levels of IFNγ. The way the measurement of an immune effector molecule is carried out is not critical for the present invention as long as the method is specific and sensitive enough.

In a first preferred embodiment of the method, step (ii) is performed as follows:
(ii) measuring the presence or elevation in the level of an immune effector molecule via the detection of its mRNA via micro-arrays or PCR or Northern blot method. In this preferred embodiment, an immune effector molecule is IFNγ.

The skilled person knows how to perform micro-arrays, PCR or Northern blot.

In a second preferred embodiment of the method, step (ii) is performed as follows:
(ii) measuring the presence or elevation in the level of an immune effector molecule. In this preferred embodiment, an immune effector molecule is IFNγ.

In this second preferred embodiment, preferably an immunoassay is carried out for measuring the presence or elevation in the presence in the level of an immune effector molecule. Wide ranges of immunoassay techniques are available such as sandwich assays, RIA (Radio Immuno Assay), ELISA (Enzyme Linked ImmunoSorbent Assay) and ELISpot (Enzyme linked immunospot assay). Several immunoassay techniques are for example described in U.S. Patent Nos. 4,016,043, 4,424,279 and 4,018,653. QuantiFERON-TB Gold (Cellestis) can also be performed (Codeluppi M et al, Transplant proc. 2006, 38:1083-1085). QuantiFERON-TB Gold is a tuberculosis (TB) specific ELISA using TB specific antigens.

A ligand to an immune effector molecule is particularly useful in detecting and/or quantitating these molecules in immunoassay. An antibody to an immune effector molecule is particularly useful. Reference to "antibody" includes parts of antibody, mammalianized (e.g. humanized) antibody, recombinant or synthetic antibody and hybrid and single chain antibody. Both polyclonal and monoclonal antibodies are obtainable by immunization with an immune effector or an antigenic fragment thereof and either type is utilizable for immunoassays. The methods of obtaining both types of sera are well known in the art. A polyclonal serum is less preferred but is relatively easily prepared by injection of a suitable laboratory animal with an effective amount of an immune effector, or antigenic part thereof, collecting serum from an animal and isolating a specific serum by any of the known immunoadsorbent techniques. Although an antibody produced by this method is utilizable in virtually any type of immunoassay, it is generally less favoured because of the potential heterogeneity of the product.

The use of a monoclonal antibody in an immunoassay is particularly preferred because of the ability to produce it in large quantities and the homogeneity of the product. The preparation of an hybridoma cell line for monoclonal antibody production derived by fusing an immortal cell line and a lymphocyte sensitized against the immunogenic preparation can be done by techniques which are well known to those who are skilled in the art.

In a preferred embodiment of the method, step (ii) is performed as follows:
(ii) measuring the presence or elevation in the level of an immune effector molecule via the addition of an antibody specific for said immune effector or antigenic fragment thereof for a time and under conditions sufficient for an antibody-effector complex to form, and then detecting said complex. In this preferred embodiment, an immune effector molecule is IFNγ.

In a more preferred embodiment of the method, an elevation in the level of an immune effector molecule of at least 10% compared to the level of an immune effector molecule measured in a negative control means that an antigen is present in the sample. An immune effector molecule is preferably detected by ELISA. More preferably, the elevation is of at least 30%, even more preferably of at least 50%, even more preferably of at least 70%, even more preferably of at least 100%, even more preferably of at least 200%, even more preferably of at least 500%, and most preferably of at least 1000%. A negative control has already been defined earlier.

In a more preferred embodiment, when an antigen is a mycobacterium, a disease is tuberculosis (TB), an immune effector molecule is IFNγ, an immune effector cell used is a TB specific polyclonal T-cells and the measurement of IFNγ is carried out by an ELISA. A TB specific polyclonal T-cell is preferably prepared as earlier defined (see paragraph entitled preparation of immune effector cells). In this preferred embodiment, an elevation in the level of IFNγ of at least 10% will mean that a mycobacterium is present in a sample. More preferably, the elevation is of at least 30%, even more preferably of at least 50%, even more preferably of at least 70%, even more preferably of at least 100%, even more preferably of at least 200%, even more preferably of at least 500%, and most preferably of at least 1000%.

ELISpot is an assay in which an immune effector cell produces an immune effector molecule which is detected at the place where an immune effector cell is located in the *in vitro* test environment and where it produces an immune effector molecule, for example at the bottom of a 96 well plate. An immune effector molecule is bound for example via coating of the bottom of the well with antibodies specific for an immune effector molecule to be detected. Subsequently an immune effector molecule may be detected using an antibody coupled to an enzyme which can be detected using a dye. As a result a spot will arise at the bottom of a well indicating the presence of an immune effector cell specific for an antigen tested. Several types of ELISpot are already known and available: WO 2005/090988, WO 2004/042396, WO 98/23960. The measurement of an immune effector molecule maybe performed by a B cell ELISpot (Quan C et al, (2006), J. Clin. Microbiol., 44:3160-3166) or by T cell ELISpot (Oxford Immunotec Ltd.). Preferably, if the disease is tuberculosis, the T cell ELISpot used is T-SPOT™.TB (Oxford Immunotec Ltd.).

In a more preferred embodiment, the presence or elevation in the level of an immune molecule is measured by ELISPOT. In this case, the presence or elevation in the level of an immune effector molecule more preferably means that at least 5% more spots are found compared to the number of spots present in a negative control. Negative control has already been defined herein. More preferably, the elevation is of at least 10%, even more preferably of at least 20%, even more preferably of at least 30%, even more preferably of at least 40%, even more preferably of at least 50%, even more preferably of at least 70%, and most preferably of at least 100%.

In a classical CMI detection method as presented in the background, a CMI response of a subject is tested via the reactivity of his own Immune cells upon a given antigen specific for a given disease. A known drawback of these classical CMI detection methods, it that an immune cells loses its capacity to mount a CMI response in whole blood after extended periods following blood draw from the subject.

In the method of the invention, one does not measure the CMI response of an immune cell of a subject. One measures the presence of an (unknown) antigen in a subject by contacting a sample from a subject with an immune cell which is already known to be responsive for a disease tested. Advantageously, the method of the invention does not need to isolate an immune cell of a subject to be tested. In the method of the invention contrary to known methods, a sample is not a (recombinant) composition comprising an unique given and known antigen indicative of the presence of a given infectious agent, medical condition or disease. An antigen tested as present in a sample is unknown. Furthermore, advantageously, only very small amounts of a sample are needed to be contacted with an immune effector cell. This method could therefore be used with a body fluid difficult to obtain in large quantities. An immune cell used does not originate from a sample from a subject wherein the presence of an antigen related to an infectious agent, medical condition or disease is being detected. As demonstrated in the examples, the method of the invention is highly specific. It is even more specific than existing immunoassays.

### Kit

In a second aspect of the invention, there is provided a kit to be used in the method of the invention. This kit is for measuring the presence or elevation in the level of an antigen in a sample, said antigen being indicative for the presence of an infectious agent, wherein the kit comprises:
a. an immune effector cell, which is capable of producing an immune effector molecule following stimulation by an antigen,
b. means for incubating said immune effector cell with a sample;
c. means for measuring the presence or elevation in the level of an immune effector molecule, wherein the presence or elevation in the level of said immune effector molecule is indicative of the presence of an antigen in a sample.

The kit is conveniently in compartmental form with one or more compartments adapted to receive a sample and to contact a sample with an immune effector cell. Generally, the kit is in a form which is packaged for sale with a set of instructions. The instructions would generally be in the form of a method for measuring the presence or elevation in the level of an antigen in a sample by,
(i) contacting an immune effector cell with a sample, wherein an immune cell is capable of producing an immune effector molecule following stimulation by an antigen,
(ii) measuring the presence or elevation in the level of an immune effector molecule, wherein the presence or elevation in the level of said immune effector molecule is indicative of the presence of an antigen in a sample.

An immune effector cell is preferably in frozen state.

Preferred means for measuring the presence or elevation in the level of an immune effector molecule were already described. As a preferred means for measuring the presence or elevation in the level of an immune effector molecule is a ligand to an immune effector molecule. More preferably, this ligand is immobilized on a solid support. The solid support may be a plate with wells, such as a microtiter plate. Preferred types of a sample, an immune effector cell, an antigen, an immune effector molecule have already been described earlier. The kit may additionally comprise medium for an immune effector cell, a detection agent, or a washing buffer to be used in the step ii) of the method of the invention. The kit may also comprise a control, such as a positive and/or a negative control. A positive control may allow the detection system of the kit to be tested. Typically a positive control mimicks the CMI response of an immune effector cell, that is to say the production of an immune effector molecule. For example, a positive control is a superantigen. A negative control is typically a composition which does not contain an antigen. The kit may also comprise a mean to take a sample to be tested.

The invention further relates to the use of the kit as defined earlier for measuring the presence or elevation in the level of an antigen in a sample, said antigen being indicative for the presence of an infectious agent.

The present invention is further illustrated by the following non-limiting examples.

In this document and in its claims, the verb "to comprise" and its conjugations is used in its non-limiting sense to mean that items following the word are included, but items not specifically mentioned are not excluded. In addition, reference to an element by the indefinite article "a" or "an" does not exclude the possibility that more than one of the element is present, unless the context clearly requires that there be one and only one of the elements. The indefinite article "a" or "an" thus usually means "at least one".

### Examples

### Experiment 1)

The method of the invention was carried out using immune effector T cells from a subject known to have been infected by tuberculosis (TB) with circulating TB specific T-cells as demonstrated with the T-spot TB (Donor 3) or cells from control subjects not infected with tuberculosis (Donors 1, not BCG vaccinated and tuberculin skin test negative, 2, BCG vaccinated and tuberculin skin test negative and 4, BCG vaccinated and tuberculin skin test positive) using pleural fluid (PF) from one subject with active tuberculosis to be tested as a sample. This sample is illustrative for the value of the technique since TB was only demonstrated after prolonged culture, whereas the cross spot was positive after 12 hours.

40 ml of PF was taken via thoracocentesis and processed immediately, As a control, immune cells present in the pulmonary fluid were also isolated. T cells from a subject known to have been earlier infected by tuberculosis (Donor 3) were isolated. In total, eight mL of citrated blood was collected in a CellPrep tube (8 mL, BD, Franklin Lanes, NJ, USA) for the isolation of peripheral blood mononuclear cells (PBMC). As a control T cells from subjects who had not been earlier infected by tuberculosis were also isolated from the blood (Donors 1, 2, 4) using the same method as described above. After blood collection, tubes were transported upright at room temperature to the laboratory. PBMC were isolated by centrifugation of CellPrep tubes for 28 minutes at 1600 g. Next, PBMC were washed in 10 mL RPMI (Gibco 21875-034, Invitrogen, The Netherlands), centrifuged for 7 min at 600 g, washed in 10 mL of RPMI and centrifuged for another 7 min at 300g. Subsequently, cells were resuspended in 1.5 mL AIM-V serum-free culture medium (Gibco, Invitrogen, The Netherlands) and counted using an automated cell counter (Coulter counter), diluted in AIM-V to a final concentration of 2.5 × 10⁶ cells/mL. PF samples were centrifuged at 900 g for 7 minutes, Supernatant was collected and the cellpellet was washed en resuspended in AIM-V serum free culture medium and counted using an automated cell counter (Coulter counter), diluted in AIM-V to a final concentration of 2.5 × 10⁶ lymphocytes/mL. After incubation of the cells with the pleural fluid and further processing the samples according to the manufacturers instructions, the number of spots was scored visually using a magnifying glass by two independent observers. In case of discrepancies, a third observer made the decisive judgement. Interpretation of results was according to the criteria defined by the manufacturer.

The experiment is presented in table 1 in which the cells obtained from donors 1-4, the cells obtained from the PF from the patient en the cell free supernatant of the PF of the patient are subjected to a classical T-SPOT. *TB* (Oxford Immunotec Ltd.) performed following the manufacturer's instructions The rows indicated with neg control are the negative controls in which the samples are not triggered with immune effector molecules. The rows indicated with pos control are the positive controls in which the samples are triggered with a T-cell superantigen to confirm whether the used T-cells can be triggered to produce IFN gamma. The rowes indicated with panel A and B are the true tests in which the samples are triggered with the TB antigens from the T-SPOT.TB kit. Results are shown in table 1. As expected one can see that the cells derived from the donors (1, 2, 4) not sensitized to TB do not show a T-cell response on exposure to the TB specific antigens as measured by the detection of the interferon gamma release. Donor 3, known to be infected with TB, shows a strong T-cell response upon triggering with the TB specific antigens, showing > 100 spots for both antigens. Cells obtained from the pleural fluid from a patient with known TB infection and TB pleurisy (PF cells) also show a strong reaction on both TB specif antigens whereas in the cell free supernatant of PF the same patient no T-cell stimulation can be observed which is consistent with the absence of immune effector cells in this test. The negative control of the PF cells experiment shows 40 spots, indicating that even without adding the TB specific antigen T-cells activation can be observed. It was our hypothesis that this is the result of the presence of both TB antigens and TB specific T-cells in the pleural fluid. In the same experiment the true invention (cross-spot) was performed using supernatant op the PF of the patient with known TB which was used as a source of TB antigen (see table 2). This preparation was used in the T-SPOT.TB assay in which the antigens of this kit were replaced with the preparation of the pleural fluid. T-cells obtained from donor 1,2, not infected with TB could not be triggered to an interferon gamma release with the PF supernatant as measured in the T-SPOT.TB test, whereas the T-cells derived from the subject with a known TB infection showed 51 and 46 spots upon stimulation with the PF supernatant in two independent observations, demonstrating that mycobacterium specific antigens are present in the PF supernatant. This observation shows that it is possible to detect mycobacterium specific antigens in PF supernatant making this invention a new method for diagnosing mycobacterial infection or TB.

Experiment 2) The method of the invention was carried out using immune effector T cells from a subject known to have been infected by EBV as demonstrated by positive IgG-EBV and negative IgM-EBV serology and from an EBV naïve subject as demonstrated by negative IgG-EBV and IgM-EBV serology. Reactivity of both donors was conformed using the superantigen as described in experiment 1. Cells from both donors were incubated with supernatant of a cell culture infected with EBV (concentration of supernatant: 90.000 copies/mL measured by EBV-PCR) and with commercially available EBV (EBV B95-8) using two dilutions (90.000 and 49.000.000 copies/mL). Results are shown in table 3. The results in experiment 2 demonstrate that sensitized PBMC's do react to EBV both harvested from the supernatant of an EBV infected cell culture and commercially obtained EBV.

**Table 1: scheme of the control experiment realized**

| test | donor I | donor 2 | donor 3 | donor 4 | PF cells | PF sup |
|---|---|---|---|---|---|---|
| neg control | 0 | 0 | 8 | 0 | 40 | 0 |
| pos control | pos | pos | pos | pos | pos | 0 |
| panel A | 0 | 0 | >100 | 1 | >100 | 0 |
| panel B | 0 | 1 | >100 | 0 | >100 | 0 |

**Table 2: scheme of the cross-spot experiment realized**

| test | donor 1 | donor 2 | donor 3 | donor 4 |
|---|---|---|---|---|
| neg control | 0 | 0 | 8 | 0 |
| pos control | pos | pos | pos | pos |
| panel PF sup | 0 | 0 | 51 | 1 |
| panel PF sup | 0 | 1 | 46 | 0 |

**Table 3: scheme of the cross-spot experiment realized using EBV as antigen**

| | Negative control | Positive control | EBV cell culture supernatant 90.000 copies/ml | EBV B95-8 90.000 copies/mL | EBV B95-8 4.900.000 copies/mL |
|---|---|---|---|---|---|
| EBV naïve donor | neg | pos | 0 | 0 | 0 |
| EBV positive donor | neg | pos | 23 | 0 | 47 |

## Claims

1. An *in vitro* method for measuring the presence or elevation in the level of an antigen in a sample, said antigen being indicative for the presence of an infectious agent, wherein said method comprises the following steps:
(i)contacting immune effector cells with the sample, wherein the immune effector cells have been primed to react by the production of an effector molecule such as a cytokine or an immunoglobuline to said antigen or are obtained from a source previously reactive/infected with said antigen,
(ii)measuring the presence or elevation in the level of said immune effector molecule, wherein the presence or elevation in the level of said immune effector molecule is indicative of the presence of the antigen in the sample.

2. A method according to claim 1, wherein the sample originates from food, feed, coating for medicine apparatus, drug.

3. A method according to claim 1 or 2, wherein the sample is a biological sample.

4. A method according to claim 3, wherein the biological sample is a human or animal body fluid.

5. A method according to claim 4, wherein the biological sample is selected from the following list: urine, faeces, blood, lymph fluid, cerebral fluid, tissue fluid, respiratory fluid including nasal and pulmonary fluid, fluids obtained by nasal or pulmonary lavage, pleural fluid and synovial fluid.

6. A method according to any one of claims 3 to 5, wherein the method is used for establishing the presence of an infectious agent.

7. A method according to claim 6, wherein the method is used for establishing the presence of a medical condition or disease, said medical condition or disease being associated with the presence of the antigen in a sample taken from a subject.

8. A method according to any one of claims 1 to 7, wherein the infectious agent is selected from the following list: bacterium, virus, fungus, parasite or prion.

9. A method according to claim 8, wherein the bacterium is a mycobacterium.

10. A method according to claim 8, wherein the virus is EBV.

11. A method according to any one of claims 1 to 10, wherein the immune cells used are T cells and/or B-cells.

12. A method according to any one of claims 1 to 11, wherein the immune effector molecule is selected from the group consisting of: IFNγ and an immunoglobuline of the IgM, IgG, IgA or IgE class.

13. A kit for measuring the presence or elevation in the level of an antigen in a sample, said antigen being indicative for the presence of an infectious agent, wherein the kit comprises:
a. immune effector cells that have been primed to react by the production of an effector molecule such as a cytokine or an immunoglobuline to said antigen or are obtained from a source previously reactive/infected with said antigen,
b. means for incubating said immune effector cells with the sample;
c. means for measuring the presence or elevation in the level of said immune effector molecule, wherein the presence or elevation in the level of said immune effector molecule is indicative of the presence of the antigen in the sample.

14. A kit according to claim 13, for measuring the presence or elevation in the level of an antigen in a sample, said antigen being indicative for the presence of a medical condition or disease.

15. Use of the kit as defined in claim 13, for measuring the presence or elevation in the level of an antigen in a sample, said antigen being indicative for the presence of an infectious agent.

16. Use of the kit according to claim 15, for measuring the presence or elevation in the level of an antigen in a sample, said antigen being indicative for the presence of a medical condition or disease.

## Patentansprüche

1. In-vitro-Verfahren zum Messen der Anwesenheit oder Erhöhung des Levels eines Antigens in einer Probe, wobei das Antigen für die Anwesenheit eines infektiösen Agens indikativ ist, wobei das Verfahren die folgenden Schritte umfasst:
(i) In-Kontakt-Bringen von Immuneffektorzellen mit der Probe, wobei die Immuneffektorzellen in der Weise aktiviert wurden, dass sie mit der Produktion eines Effektormoleküls, wie zum Beispiel einem Cytokin oder einem Immunglobulin, gegen das Antigen reagieren, oder aus einer Quelle gewonnen werden, welche zuvor mit dem Antigen reagierte/infiziert wurde,
(ii) Messen der Anwesenheit oder Erhöhung des Levels des Immuneffektormoleküls, wobei die Anwesenheit oder Erhöhung des Levels des Immuneffektormoleküls für die Anwesenheit des Antigens in der Probe indikativ ist.

2. Verfahren gemäß Anspruch 1, wobei die Probe aus Lebensmittel, Futtermittel, Beschichtung von medizinischen Apparaten, Arzneimittel stammt.

3. Verfahren gemäß Anspruch 1 oder 2, wobei die Probe eine biologische Probe ist.

4. Verfahren gemäß Anspruch 3, wobei die biologische Probe eine humane oder tierische Körperflüssigkeit ist.

5. Verfahren gemäß Anspruch 4, wobei die biologische Probe aus der folgenden Liste ausgewählt ist: Urin, Kot, Blut, Lymphflüssigkeit, Hirnflüssigkeit, Gewebeflüssigkeit, respiratorische Flüssigkeit, beinhaltend nasale und pulmonale Flüssigkeit, Flüssigkeiten, gewonnen durch nasale oder pulmonale Lavage, Pleuralflüssigkeit und Synovialflüssigkeit.

6. Verfahren gemäß einem der Ansprüche 3 bis 5, wobei das Verfahren zur Ermittlung der Anwesenheit eines infektiösen Agens verwendet wird.

7. Verfahren gemäß Anspruch 6, wobei das Verfahren zur Ermittlung der Anwesenheit eines medizinischen Zustands oder einer Krankheit verwendet wird, wobei der medizinische Zustand oder die Krankheit mit der Anwesenheit des Antigens in einer Probe, welche von einem Subjekt genommen wurde, assoziiert ist.

8. Verfahren gemäß einem der Ansprüche 1 bis 7, wobei das infektiöse Agens aus der folgenden Liste ausgewählt ist: Bakterium, Virus, Pilz, Parasit oder Prion.

9. Verfahren gemäß Anspruch 8, wobei das Bakterium ein Mycobakterium ist.

10. Verfahren gemäß Anspruch 8, wobei das Virus EBV ist.

11. Verfahren gemäß einem der Ansprüche 1 bis 10, wobei die verwendeten Immunzellen T-Zellen und/oder B-Zellen sind.

12. Verfahren gemäß einem der Ansprüche 1 bis 11, wobei das Immuneffektormolekül ausgewählt ist aus der Gruppe, bestehend aus: IFNγ und einem Immunglobulin der IgM-, IgG-, IgA- oder IgE-Klasse.

13. Kit zum Messen der Anwesenheit oder Erhöhung des Levels eines Antigens in einer Probe, wobei das Antigen für die Anwesenheit eines infektiösen Agens indikativ ist, wobei das Kit:
a. Immuneffektorzellen, welche in der Weise aktiviert wurden, dass sie mit der Produktion eines Effektormoleküls, wie zum Beispiel einem Cytokin oder einem Immunglobulin, gegen das Antigen reagieren, oder aus einer Quelle gewonnen werden, welche zuvor mit dem Antigen reagierte/infiziert wurde;
b. Mittel zum Inkubieren der Immuneffektorzellen mit der Probe;
c. Mittel zum Messen der Anwesenheit oder Erhöhung des Levels des Immuneffektormoleküls, wobei die Anwesenheit oder Erhöhung des Levels des Immuneffektormoleküls für die Anwesenheit des Antigens in der Probe indikativ ist,
umfasst.

14. Kit gemäß Anspruch 13 zum Messen der Anwesenheit oder Erhöhung des Levels eines Antigens in einer Probe, wobei das Antigen für die Anwesenheit eines medizinischen Zustands oder einer Krankheit indikativ ist.

15. Verwendung des Kits gemäß Anspruch 13 zum Messen der Anwesenheit oder Erhöhung des Levels eines Antigens in einer Probe, wobei das Antigen für die Anwesenheit eines infektiösen Agens indikativ ist.

16. Verwendung des Kits gemäß Anspruch 15 zum Messen der Anwesenheit oder Erhöhung des Levels eines Antigens in einer Probe, wobei das Antigen für die Anwesenheit eines medizinischen Zustands oder einer Krankheit indikativ ist.

## Revendications

1. Procédé *in vitro* servant à détecter la présence d'un antigène dans un échantillon ou à mesurer la hausse de la concentration d'un antigène dans un échantillon, ledit antigène étant un indice de la présence d'un agent infectieux, lequel procédé comporte les étapes suivantes :
i) mettre l'échantillon en contact avec des cellules immunitaires effectrices qui ont été sensibilisées pour réagir audit antigène en produisant une molécule effectrice, telle une cytokine ou une immunoglobuline, ou qui ont été obtenues à partir d'une source antérieurement réactive vis-à-vis dudit antigène ou préalablement infectée avec ledit antigène ;
ii) et détecter la présence de ladite molécule immuno-effectrice ou mesurer la hausse de la concentration de cette molécule, la présence de ladite molécule immuno-effectrice ou la hausse de la concentration de cette molécule étant un indice de la présence de l'antigène dans l'échantillon.

2. Procédé conforme à la revendication 1, dans lequel l'échantillon provient d'un aliment, d'une nourriture pour animaux, d'un revêtement d'appareil médical ou d'un médicament.

3. Procédé conforme à la revendication 1 ou 2, dans lequel l'échantillon est un échantillon biologique.

4. Procédé conforme à la revendication 3, dans lequel l'échantillon biologique est un fluide corporel humain ou animal.

5. Procédé conforme à la revendication 4, dans lequel l'échantillon biologique est choisi dans la liste suivante : urine, fèces, sang, lymphe, liquide cérébral, liquide interstitiel, liquides de l'appareil respiratoire, y compris les sécrétions nasales et les sécrétions pulmonaires, liquides obtenus par lavage du nez ou des poumons, liquide pleural et synovie.

6. Procédé conforme à l'une des revendications 3 à 5, lequel procédé est utilisé pour établir la présence d'un agent infectieux.

7. Procédé conforme à la revendication 6, lequel procédé est utilisé pour établir la présence d'un état ou trouble médical, lequel état ou trouble médical est associé à la présence de l'antigène dans un échantillon prélevé chez un sujet.

8. Procédé conforme à l'une des revendications 1 à 7, pour lequel l'agent infectieux est choisi dans la liste suivante : une bactérie, un virus, un champignon inférieur, un parasite et un prion.

9. Procédé conforme à la revendication 8, dans lequel la bactérie est une mycobactérie.

10. Procédé conforme à la revendication 8, dans lequel le virus est le virus Epstein-Barr (EBV).

11. Procédé conforme à l'une des revendications 1 à 10, dans lequel les cellules immunitaires utilisées sont des lymphocytes T et/ou des lymphocytes B.

12. Procédé conforme à l'une des revendications 1 à 11, pour lequel la molécule immuno-effectrice est choisie parmi l'interféron gamma et une immunoglobuline de classe IgM, IgG, IgA ou IgE.

13. Trousse servant à détecter la présence d'un antigène dans un échantillon ou à mesurer la hausse de la concentration d'un antigène dans un échantillon, laquelle trousse comprend :
a) des cellules immunitaires effectrices qui ont été sensibilisées pour réagir audit antigène en produisant une molécule effectrice, telle une cytokine ou une immunoglobuline, ou qui ont été obtenues à partir d'une source antérieurement réactive vis-à-vis dudit antigène ou préalablement infectée avec ledit antigène ;
b) des moyens permettant de faire incuber ces cellules immunitaires effectrices avec l'échantillon ;
c) et des moyens permettant de détecter la présence de ladite molécule immuno-effectrice ou de mesurer la hausse de la concentration de cette molécule, la présence de ladite molécule immuno-effectrice ou la hausse de la concentration de cette molécule étant un indice de la présence de l'antigène dans l'échantillon.

14. Trousse conforme à la revendication 13, servant à détecter la présence d'un antigène dans un échantillon ou à mesurer la hausse de la concentration d'un antigène dans un échantillon, ledit antigène étant un indice de la présence d'un état ou trouble médical.

15. Utilisation d'une trousse conforme à la revendication 13 pour détecter la présence d'un antigène dans un échantillon ou mesurer la hausse de la concentration d'un antigène dans un échantillon, ledit antigène étant un indice de la présence d'un agent infectieux.

16. Utilisation, conforme à la revendication 15, d'une trousse pour détecter la présence d'un antigène dans un échantillon ou mesurer la hausse de la concentration d'un antigène dans un échantillon, ledit antigène étant un indice de la présence d'un état ou trouble médical.
